(19) Europäisches Patentamt
European Patent Office
Office européen des brevets

(11) **EP 4 492 018 A1**

(12) **EUROPEAN PATENT APPLICATION**

(43) Date of publication:
**15.01.2025 Bulletin 2025/03**

(21) Application number: **23184785.6**

(22) Date of filing: **11.07.2023**

(51) International Patent Classification (IPC):
**G01H 9/00** (2006.01)      **A61B 5/00** (2006.01)
**G01N 21/17** (2006.01)      **G02B 6/125** (2006.01)

(52) Cooperative Patent Classification (CPC):
**G01H 9/004; A61B 5/0097;** A61B 5/6852;
G02B 6/125

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Nederlandse Organisatie voor
toegepast-natuurwetenschappelijk Onderzoek
TNO
2595 DA 's-Gravenhage (NL)**

(72) Inventors:
• **HARMSMA, Peter Johan
  2595 DA 's-Gravenhage (NL)**

• **QUESSON, Benoit André Jacques
  2595 DA 's-Gravenhage (NL)**
• **VAN NEER, Paul Louis Maria Joseph
  2595 DA 's-Gravenhage (NL)**
• **VAN DER HEIDEN, Maurits Sebastiaan
  2595 DA 's-Gravenhage (NL)**
• **ALTMANN, Robert Karl
  2595 DA 's-Gravenhage (NL)**
• **PIRAS, Daniele
  2595 DA 's-Gravenhage (NL)**

(74) Representative: **V.O.
P.O. Box 87930
2508 DH Den Haag (NL)**

(54) **ON-CHIP OPTO-ACOUSTIC INTERFEROMETERS**

(57) Aspects of the present disclosure relate to an on-chip opto-acoustic transducer element and to an ultrasound receiver as well as an imager assembly comprising said transducer element, and to a method of manufacturing.

The transducer element comprises a membrane (5) configured to deflect in response to an acoustic pressure wave; a primary photonic waveguide (WG-1, 1) disposed at the membrane, and an auxiliary photonic waveguide (WG-2, 2) disposed at the membrane, wherein the primary waveguide and the auxiliary photonic waveguide follow a respective curved primary and auxiliary curved trajectory over the membrane which extends over a respective first zone (z1) and a respective second zone (z2) of the membrane providing opposite sign expansion/contraction to the due to a deflection of the membrane in response to the acoustic pressure wave.

FIG 2B

**Description**

TECHNICAL FIELD AND BACKGROUND

The present disclosure relates to an on-chip opto-acoustic transducer element for converting an acoustic pressure wave into a modulation of a property of light. The present disclosure further relates to an ultrasound receiver, an imager assembly comprising said transducer element and to a method of manufacturing.

The basic principle is that one or more acoustic source sends out an ultrasound signal into a sample, e.g. tissue. Ultrasound receivers are used to detect an output signal in the form of amplitude and/or phase. Usually in a linear/-curved/phased array for example to construct a 2D image, Tomography allows the construction of a three-dimensional image of the tissue. Typically, multiple emitters and/or receivers are used, and advanced signal optimization and processing to optimize results.

Opto-acoustic receivers comprising a resonator supporting an optical wave guide for converting an acoustic pressure wave into a modulation of a property of light are known. The mechanical resonance frequency of the receiver is typically matched to the frequency of the ultrasound signal, so that mechanical amplification of the modulation can be obtained.

The minimal achievable noise equivalent pressure (NEP) of Mach Zehnder Interferometer based Integrated Photonics Ultrasound Transducers (IPUTs) is limited by foundry and material platform limitations regarding optical wave guide geometries (minimal bend radius, optical wave guide losses).

This limits the amount of optical waveguide loops which fit on a particular membrane. Even if it does fit, for small membranes the optical waveguides are not necessarily located on optimal sites on the membrane.

The minimal bend radius of the optical wave guides also limits the minimal membrane diameter and therefore upper resonance frequency.

EP3851815A1 discloses a ring resonator and a MZI interferometer comprising a sensing waveguide that extends over multiple membranes.

WO2021145766A1 discloses a photonic device having a sensing waveguide that extends across a plurality of membranes.

WO2021145766A1 relates to a photonic integrated device that comprises a substrate, a plurality of mechanical resonator structures on a surface of the substrate, exposed to receive sound waves from outside the device; a plurality of sensing optical waveguides, each sensing optical waveguide at least partly mechanically coupled to at least one of the mechanical resonator structures, or a sensing optical waveguide that is at least partly mechanically coupled to all of the mechanical resonator structures.

US2020319019A1 discloses a ring resonator comprising an optical waveguide that extends over a plurality of membranes.

While the known devices can provide some benefits there remains a desire for sensors having increased sensitivity, reduced dimension and/or for operating at comparatively higher acoustic frequencies.

SUMMARY

Aspects of the present disclosure relate to an on-chip integrated opto-acoustic transducer element; an on-chip ultrasound receiver device comprising the transducer element; and to an ultrasound imager assembly comprising the transducer element as disclosed herein. Further aspects relate to a process of manufacturing an opto-acoustic transducer element, preferably the opto-acoustic transducer element as disclosed herein. Advantageously the opto-acoustic transducer element and/or the receiver can be embodied as an on chip device, e.g. a fully integrated on chip device.

The opto-acoustic transducer can be used to advantage for converting an acoustic pressure wave into a modulation of a property of light, e.g. for the detection of ultrasound and/or for the construction of an image, e.g. as in ultrasound imaging devices such as medical ultrasound imagers.

As will be explained in more detail in the specification herein below the presented opto-acoustic transducer element can particularly be used for opto-acoustic imaging with one or more of: improved sensitivity, improved resolution, decreased form factor, and for imaging at comparatively higher acoustic frequencies than known devices with a comparable signal to noise level.

The on-chip integrated opto-acoustic transducer element according to the present disclosure comprises at least a carrier substrate having a membrane layer extending over an aperture in a surface of the substrate. The aperture may be fully etched through the substrate, or may be a recess. The membrane is configured to deflect in response to a received acoustic pressure wave, typically an external acoustic pressure wave. The transducer further comprises at least a primary photonic waveguide disposed at the membrane, and an auxiliary photonic waveguide disposed at the membrane, wherein the primary waveguide and the auxiliary photonic waveguide follow a respective primary and auxiliary trajectory which extend over a respective first zone (z1) of the membrane and a respective second zone (z2) of the membrane providing opposite sign expansion/contraction of the waveguide due to a deflection of the membrane in response to the acoustic pressure wave. Positioning the primary photonic waveguide and the auxiliary photonic waveguide along zones providing opposite sign expansion/contraction of the waveguide due to a deflection of the membrane can effectively double an output (e.g. a phase difference or light intensity) of the transducer element. As discussed herein the waveguide is typically a so-called single mode waveguide. It will be appreciated that the concepts as disclosed herein can be applied to multimode waveguides, unless clear from context or stated to the contrary,

The opto-acoustic transducer can be used to advantage for converting an acoustic pressure wave into a modulation of a property of light, e.g. for the detection of ultrasound and/or for the construction of an image, e.g. as in ultrasound imaging devices such as medical ultrasound imagers. The transducer can be configured to operate according to known sensing principles. For example, the primary and auxiliary waveguide can be arranged according to interference based sensing principles (e.g. as in a Mach Zehnder interferometer configuration) or according to optical resonance principles (e.g. as in in a ring resonator).

Advantageously, the properties of the waveguides (e.g. length, shape, etc.) can be less restricted by the properties/dimensioning of the membrane (e.g. shape, dimension, resonance properties). Particularly, the present disclosure allows guiding the waveguide along comparatively smaller membranes (higher resonance frequencies) than conventional resonators wherein the waveguide follows a spiraling trajectory at the membrane.

In a preferred variation the primary and auxiliary waveguide follow a curved trajectory each having a radius of curvature larger than a radius of curvature of the membrane. In such configurations, the membrane can be configured to resonate at high resonance frequencies.

Preferably, the waveguides follow a largely (>50%) curved trajectory at the membrane, more preferably the trajectories follow predominately (>75%) curved trajectory where in contact with the membrane, or even a trajectory that is curved over >95% of its length , e.g. up to essentially its entire length (>99% or even 100%), at the membrane. Inventors found that, as compared to straight (linear) trajectories curved waveguides can advantageously experience an improved sensitivity (better signal to noise (S/N)). Without wishing to be bound by theory it is believed that the improved S/N can be attributed to a larger total integrated absolute effect (positive or negative) on the light path properties within the waveguide due to deflection of the membrane as compared to waveguides following a straight trajectory.

In a preferred embodiment, the waveguides extend across a plurality of membranes. The membrane can be arranged in an array e.g. 2D-array or any other appropriate configuration, (e.g. a pattern following a curvature of the waveguide). Extending the waveguides across a plurality of membranes can improve an overall sensitivity.

In another or further preferred embodiment, the primary and auxiliary curved trajectory each have a radius of curvature larger than a radius of curvature of the membrane, at least where in contact with the membrane. Extending the waveguides across a plurality of membranes can be especially relevant for small membranes (for high frequency imaging) which, due to their small dimension, cannot accommodate long waveguides (e.g. a spiral shaped section).

In a preferred variation, the membranes are provided in an array spanning an area having a size equal or smaller than half the wavelength of the acoustic wave squared. If the pitch is larger than 0.5*wavelength, the transducer may not adhere to the spatial sampling criterion, which may impart sensitivity to sound incoming from unwanted directions.

In some preferred embodiments, the first and second zones are at opposing sides of the membrane across a neutral bending plane (N) of the resonator membrane. Positioning the primary photonic waveguide and the auxiliary photonic waveguide at opposing sides of the membrane can advantageously provide a configuration wherein light propagating within the waveguides experience an opposite signal response, for each out of plane deflection of the membrane (e.g. a fundamental resonance mode). Positioning the primary photonic waveguide and the auxiliary photonic waveguide along opposing sides of the membrane zones can effective provide an up to double interferometric output of the transducer element.

In other preferred variations, the first zone and the second zone are laterally spaced along the same side of the membrane at positions having an out of phase deflection in accordance with a higher harmonic resonance mode of the membrane. For example, in accordance with positions having maximum deflection at a second order (harmonic) resonance of the membrane. Positions having an out of phase deflection in accordance with a higher harmonic resonance mode can be determined experimentally for a given membrane (e.g. by optical microscopy and/or modelling). Alternatively, or in addition, having an out of phase deflection, e.g. $n \times \pi$ phase shift, in accordance with a higher harmonic resonance mode can be determined in accordance with a method as disclosed herein and/or by providing anisotropy within the membrane and/or an anisotropy between adjacent membranes. Positioning the primary photonic waveguide and the auxiliary photonic waveguide at positions experiencing an out of phase deflection in accordance with a higher harmonic resonance mode of the membrane can up to double an interferometric output of the transducer element.

In other or further preferred embodiments, at least one of the primary curved trajectory and auxiliary curved trajectory extend along a plurality of membranes, each membrane having a lateral dimension smaller than a maximum radius of curvature of the primary curved and auxiliary curved trajectory at the membrane. Preferably, the plurality of membranes are organized in an area smaller than half of the wavelength ($\lambda$p) of the acoustic pressure wave squared, i.e. ($\lambda$p/2)$^2$.

In some embodiments, the membrane can be provided with an anisotropic pre-stress. In addition to providing an additional handle on tuning a resonance frequency and/or Q-factor of the membrane, provision of an anisotropic pre-stress can advantageously realize a preorientation of higher order resonance modes of the membrane (e.g. $2^{nd}$ order resonance). Preorientation can advantageously ease a manufacturing of the device as the preorientation can be used as a priori input for the relative and/or absolute position/orientation of the waveguide(s) at the membrane.

In another or further preferred embodiment, preorientation can be realized by providing (or inducing) a membrane having a crystalline anisotropy.

In yet other or yet further preferred embodiments, preorientation can be realized by providing a coupled membrane array, e.g. an array of membranes with anisotropic spacing between adjacent ones. In a preferred variation the membrane is comprised in an array of membranes whereby a spacing between adjacent membranes along orthogonal directions within the array differs by an amount providing directional coupling of second or higher order harmonic resonance modes of the membranes. For example, the array can be a two dimensional grid (e.g. an mxn grid) whereby the spacing along columns differs from the spacing along rows.

Alternatively, or in addition, predictable directionality of membrane resonance modes can be provided by providing a specific shape having anisotropic resonant behavior (e.g. elliptical, rectangular, or other shapes as known in the field).

In some embodiments, one or more of the waveguide can physically, or at least conceptually, be considered to comprise a configuration having a first waveguide portion, a second waveguide portion, and a waveguide coupler. The waveguide coupler is disposed between the first and the second waveguide portions and interconnects the portions to provide an extended light path. In this way the two waveguide portions can effectively be interpreted as a continuous elongated waveguide. For example, the waveguide can be embodied as a sensing arm of interferometric measurement configuration, including but not limited to a so-called Mach-Zender interferometric configuration (MZI) or a Fabry-Perot-interferometer. Alternatively or additionally the waveguide can be embodied as part of a ring resonator measurement configuration. The interconnected configuration as disclosed herein advantageously allows for increased sensitivity for a membrane as compared to devices having a conventional waveguide for the same membrane.

In some embodiments, the coupler can be realized as what is also referred to herein as a merged coupler, or a merged interconnect. In this case there is provided an on-chip opto-acoustic transducer element for converting an acoustic pressure wave into a modulation of a property of light, the transducer element comprising: a membrane extending over an aperture in a surface of the carrier substrate; and a waveguide layer structure disposed at the membrane, the waveguide layer structure comprising a waveguide providing a light path extending between an input and an output, the waveguide comprising: a first waveguide portion; a second waveguide portion, and wherein the first waveguide portion continues into the second waveguide portion under an abrupt angle forming a merged interconnect. The interconnect terminates at a mirror element configured to reflect light traveling along one of the first and the second portion section to the other. In a preferred variation, the mirror element can be an upstanding sidewall of the waveguide, whereby the sidewall and waveguide portions are arranged to reflect a propagating wave by total internal reflection.

Accordingly, in some embodiments the transducer element as disclosed, wherein at least one of the primary photonic waveguide and the auxiliary photonic waveguide comprises a first curved waveguide section and a second curved waveguide section that are interconnected by a coupler, wherein the first waveguide section continues into the second waveguide section under an abrupt angle forming a merged interconnect, the interconnect terminating at a mirror element configured to reflect light traveling along one of the first and the second section to the other, forming the coupler. Among others this advantageously increase sensitivity. In some preferred embodiments, the conventional S-shaped coupler can be entirely dispensed with while realizing a comparable or even increased overall length of the waveguide along a given membrane. In addition the present disclosure can realize beneficial waveguide routing along the membrane without in plane waveguide crossings.

In some embodiments the primary photonic waveguide and the auxiliary photonic waveguide are spiral shaped, each having a first spiral section and a second spiral waveguide section that spiral inwardly, respectively outwardly, about a center of the first zone, respectively the second zone. In a preferred variation the first and the second spiral waveguide section are interconnected by the coupler element. As compared to a spiral comprising a conventional S-Shaped section the interconnected coupler can realize a more dense coverage along the membrane.

According to a further aspect there is provided an ultrasound receiver device. Advantageously the opto-acoustic transducer element and/or the opto-acoustic receiver can be embodied as an on-chip device, e.g. a fully integrated on-chip device. In a preferred embodiment, the receiver comprises: an on-chip opto-acoustic transducer element according to the present disclosure. The receiver further includes: at least one light input and one or more light output operably connected to the primary photonic waveguide and the auxiliary photonic waveguide, and readout circuitry including one or more light detector operably connected to the one or more light output and configured to provide as, electrical signals, an output based on a detected light intensity and/or phase shift.

According to a further aspect an ultrasound imager system is provided. The system comprises: the on-chip ultrasound receiver element as disclosed herein; an ultrasound transmitter; a light source operably connected to the one or more on-chip ultrasound receiver element, a processing unit configured for controlling the ultrasound source, photo acoustic receiver, the light source and the readout circuitry, and a signal processor for construction an image using the output of the readout circuitry.

Further aspects of the present disclosure relate to a method of manufacturing an opto-acoustic transducer element for converting an acoustic pressure wave into a modulation of a property of light in the transducer element, the method comprising: providing a membrane extending over an aperture or recess in a surface of a carrier substrate, the membrane layer configured to deflect in response to an acoustic pressure wave; disposing a primary photonic waveguide and an auxiliary photonic waveguide at the membrane, wherein the primary and the auxiliary photonic waveguide are disposed along a respective curved primary and auxiliary curved trajectory over the membrane which extend over a respective first zone of the membrane and a respective second zone of the membrane providing opposite sign expansion/contraction due to a deflection of the membrane in response to the acoustic pressure wave.

In a preferred embodiment, the method further comprises a step of determining characteristics of the primary and auxiliary trajectory. The parameter space can comprise one or more of a length, a radius of curvature, and a relative position of the first and the second trajectory for a given membrane. The determining can comprise modelling process, e.g. a finite element modelling, comprising: maximizing a pathlength difference between the primary and auxiliary waveguide by, for both the primary and auxiliary waveguide, integrating local contributions to contraction/expansions over the length of the waveguide due to the deflection of the membrane.

In another or further preferred embodiment, the method further comprises a step of fixing an orientation of a second or higher order harmonic resonance mode of the membrane by one or more of: providing an anisotropic pre-stress to the membrane; forming the membrane of a layer having a crystalline anisotropy; and/or providing the membrane in an array of membranes whereby a spacing between adjacent membrane along orthogonal directions within the array differs by an amount providing directional coupling.

BRIEF DESCRIPTION OF DRAWINGS

These and other features, aspects, and advantages of the apparatus, systems and methods of the present disclosure will become better understood from the following description, appended claims, and accompanying drawing wherein:

FIG 1 illustrates an ultrasound receiver concept;
FIGs 2A and 2B provide a top view image and a partial cross-section side view of an ultrasound imager assembly;
FIGs 3A, 3B, and 3C provide schematic views of transducer elements;
FIGs 4A, 4B and 5 provide schematic views of transducer elements;
FIGs 6A, and 6B provide schematic views of a transducer element;
FIGs 6C provides schematic views of a dual sided transducer element;
FIGs 7 and 8 illustrate zones of a membrane providing opposite sign expansion/contraction to the due to membrane deflection;
FIG 9 and 10 provide a schematic views of merged interconnects;
FIGs 11 and 12 provide schematic views of transducer elements;
FIG 13 schematically illustrates a method of manufacturing,
FIG 14A, 14B, 16 and 17 provide experimental details, and
FIGs 15A and 15B detail aspects of a mode-coupled array of membranes.

DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

The present disclosure in a broad sense relates to ultrasound receivers. In particular the disclosure aims to provide an improved sensitivity and/or to enable imaging at comparatively higher acoustic frequencies over known IPUTs. The acoustic operating frequency can advantageously be $\geq$ 10 MHz e.g. $\geq$ 100 MHz, A primary application of ultrasound receivers is in medical imaging systems. For example, for imaging of (human) tissue and organs. The basic principle involves that an acoustic source sends out an ultrasound signal into the tissue. The signal is picked up by a receiver element. The receiver includes a transducer for converting an acoustic pressure wave into a modulation of a property of light. The amplitude and/or phase of light as detected by an ultrasound receiver can allow the construction of a three-dimensional image. Multiple emitters and/or receivers can be used, in combination with advanced signal optimization and processing to optimize results. Operating at higher frequencies can advantageously increase imaging resolution.

An objective of the present disclosure is to increase the receiver sensitivity per unit area. Increasing the sensitivity can advantageously improve a signal-to-noise ratio and therefore a better image quality, which can be particularly relevant in view of medical regulations on the limitation of the emitted acoustic energy of the acoustic emitter. Alternatively, or in addition, increasing sensitivity can advantageously compensate for attenuation of the ultrasound signals, e.g. during propagation through tissue, so that features can be imaged from a comparatively increased distance. Alternatively, or in addition, increased sensitivity can advantageously improve imaging resolution by enabling imaging at comparatively shorter acoustic wavelengths, where the ultrasound attenuation is generally higher.

At the same time increased sensitivity per unit area allows manufacturing of comparatively smaller receivers, which in turn enables miniaturization of receivers for a given signal to noise ratio. A small receiver can, for example, be fitted into catheters for in-vivo imaging. Typically, the size of the transducer aperture/recess is determined by the acoustic wavelength (spatial sampling). So for an array transducer, the size of the individual elements would become smaller with IPUTs in accordance with the present disclosure, but the pitch can still be the same.

Additionally, a mechanical resonance frequency of the receiver is typically matched to the frequency of the ultrasound signal, so that mechanical amplification is obtained. Short acoustic wavelengths for high-resolution imaging thus benefit from small receivers having high mechanical resonance frequencies.

The present disclosure pertains to opto-acoustic ultrasound receivers. In such receivers, a membrane is excited by the ultrasound signal. On or within the membrane are photonic waveguide-based devices provided such as ring resonators or interferometer devices such as Mach Zehnder Interferometers (MZIs). The optical wavelength response of the photonic device is altered as the ultrasound signal deflects the membrane. The photonic device transmission is generally observed at one optical wavelength, and the transmitted optical power is representative for the ultrasound signal.

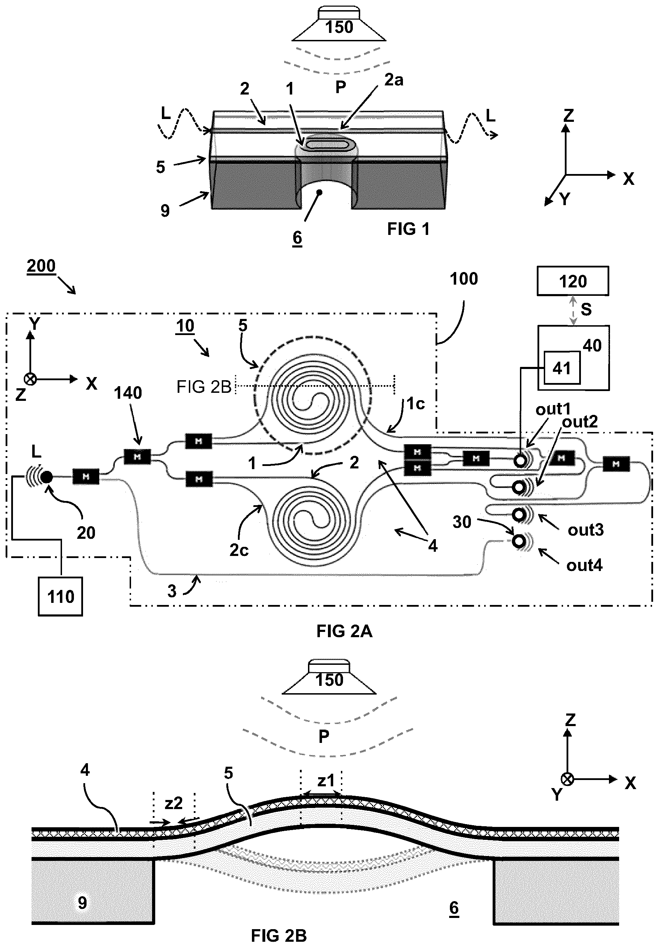

FIG 1 illustrates an ultra sound receiver concept redrawn from a publication by S.M. Leinders, et al in Nature Scientific Reports, 14328, (2015). The receiver comprises a carrier substrate 9 and a membrane layer 5. The membrane spans across an aperture (or recess) 6 in the substrate. The membrane deflects in response to an acoustic pressure wave (P). The mechanical resonance frequency of the membrane is matched to the frequency of the ultrasound signal as emitted by emitter 150.

In addition the device comprises a pair of optical waveguides. In the embodiment as shown the waveguides include a primary waveguide 1 embodied as a ring resonator and an auxiliary waveguide 2 including a coupling section 2a. Alternatively, the waveguide can be embodied as an interferometer. For example, a Mach Zehnder configuration as illustrated in 2A and 2B. For interferometers the waveguide structure 4 typically also includes at least a primary waveguide 1 and an auxiliary waveguide 2, whereby the primary waveguide 1 (also referred to as sensing waveguide) extends along the membrane and the auxiliary waveguide 2 (typically not at the membrane) is referred to as a reference waveguide.

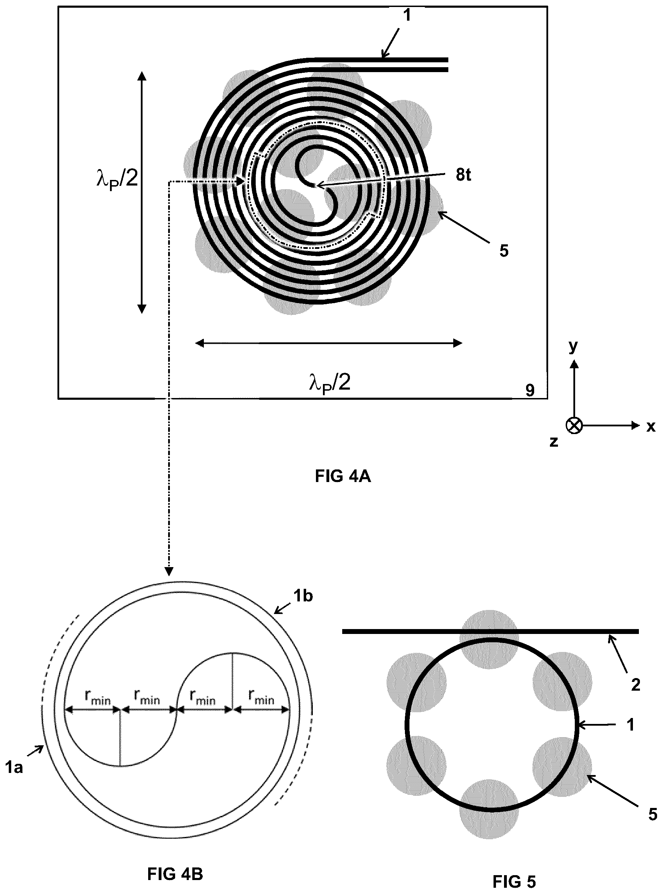

To increase a response the sensing waveguide 1 can be provided as a double spiral, e.g. as shown in FIG 2A. In order to avoid in-plane waveguide crossings (which would lead to spurious reflections) the waveguide makes a 180 degree turn (see e.g. Figure 4A and B). As such spiral can conceptually be interpreted as including a first waveguide portion 1a and a second waveguide portion 1b that are interconnected at a coupler element 8 and that combinedly provide a light path extending between an input 20 and an output 30.

As illustrated in FIG 4A and in more detail in FIG 4B, the conventional turn (8t) occupies a large area of the membrane due to the minimum bending radius ($r_{min}$) of the waveguide, below which optical losses within the waveguide rapidly increase. This large area does not add to sensitivity, and sets a limit to the minimum membrane size and therefore to the maximum mechanical resonance frequency.

As indicated in FIG 4A, the plurality of the membranes can be organized at the carrier substrate 9 in an area smaller than half of the wavelength ($\lambda$p) of the acoustic pressure wave squared.

Inventors now found that performance can be increased in configurations wherein the primary waveguide and the auxiliary photonic waveguide follow a respective primary and auxiliary trajectory over the membrane which extend over a respective first zone z1 of the membrane and a respective second zone z2 of the membrane providing opposite sign expansion/contraction of the waveguide due to a deflection of the membrane in response to the acoustic pressure wave (see e.g. Figs 2B, 7, and 8).

FIGs 2A and 2B provide a top view image and a partial cross-section side view illustrating aspects of an ultrasound imager assembly 200 according to the present disclosure. The imager assembly 200 includes a receiver device 100, an on-chip transducer element 10, and one or more of a light source 110, a readout circuitry 40, and a processing unit 120. The ultrasound imager assembly 200 can also include one or more ultrasound transmitter 150 (see figure 2B). A plurality of waveguide couplers/splitters 140 (also 'M' in Figs), as known in the field, are provided to interconnect the respective waveguides.

The readout circuitry 40 is configured to provide, as electrical signals, an output (S) based on a detected light intensity and/or phase shift. The processing unit 120 is configured for processing the output. The readout circuitry 40 includes light sensors 41 (only one indicated for clarity) operably connected to the one or more output 30 and configured to provide as, electrical signals, an output (S) based on a detected light intensity and/or phase shift. In the embodiments as shown light output 1 and 2 (out 1 and 2) correspond to a phase difference interference between spiraling waveguides 1 and 2. Light output 3 (out 3) corresponds to the output of the single loop waveguides 1c and 2c and output 4 (out 4) corresponds to a power tap output and can be used to optimize a power output of light source 110.

The transducer element comprises at least a membrane 5 extending over an aperture 6 in a surface of a carrier substrate 9 and a waveguide layer structure 4 disposed at the membrane. Preferably, the membrane 5 is configured to have a resonance mode (typically a principal resonance mode) within a frequency range of the emitter for a desired medium to be imaged (e.g. water, tissue).

The waveguide layer structure 4 comprises at least a primary waveguide 1 providing a light path extending between an input 20 and an output 30.

Note that, for ease of understanding, the auxiliary photonic waveguide 2 is illustrated as being located outside the membrane. As detailed herein for embodiments according to the present disclosure the primary photonic waveguide and auxiliary photonic waveguide are both disposed along the same membrane or multiple membranes at positions that, during operation, provide opposite sign expansion/contraction of the waveguide due to a deflection of the membrane in response to the acoustic pressure wave.

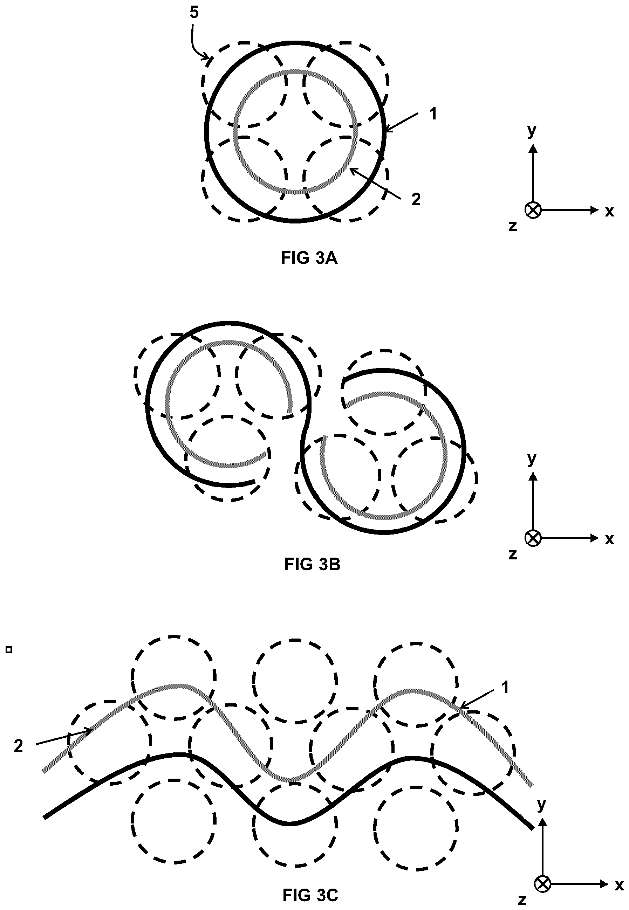

Also note that the primary and reference waveguide are depicted as conventional spirals having a 180 degree turn at the center. While the primary and/or secondary photonic waveguide may take such configuration in some embodiments, a double spiral is not essential. In fact some preferred embodiments other layouts are preferred. Details as to the positioning and shape of the waveguide trajectories will be explained in further detail with reference to FIGs 3 to 17.

In general, a minimal achievable noise equivalent pressure (NEP) of Interferometer IPUTs (Integrated Photonic Ultrasound Transducer) can be understood as being limited by foundry and material platform limitations regarding optical wave guide geometries (minimal bend radius, optical wave guide losses), which limits the amount of optical waveguide loops that can be fitted on a particular membrane. On top of that, even if a specific geometry would fit performance may be limited due to optical wave guides being located on nonideal sites on the membranes. This is especially relevant for membranes configured to operate at high resonance frequencies, e.g. small membranes. In addition, the minimal bend radius of the optical wave guides can limit a minimal membrane diameter and therefore upper resonance frequency.

In some embodiments according to the present disclosure there is provided a transducer element wherein at least one of the primary curved trajectory and auxiliary curved trajectory extend along a plurality of the membranes, each membrane having a lateral dimension smaller than a radius of curvature of the primary curved and auxiliary curved trajectory at the membrane.

Figs 3A, 3B, 3C, 4A, and 5 illustrate exemplary embodiments wherein at least one, preferably both of the primary and secondary photonic waveguide 1,2 extend along a plurality of membranes. As shown the membrane can have a radius of curvature that is smaller than a radius of curvature of the waveguide. For example, FIGs 3A-3C illustrate embodiments wherein the respective waveguides are routed along a plurality of membranes.

In generic terms it was found that the noise equivalent pressure (NEP) - or the lowest pressure a sensor can resolve - depends on the membrane diameter, it's elastic properties, the total length of the optical waveguide, losses in said waveguide. For example, for a Mach Zender interferometers comprising multiple spirals each located on a different membrane it was found that it can hold that:

$$NEP_{MZI} = \frac{1}{m} \cdot \frac{8\sqrt{2}}{3\pi^2} \cdot \sqrt{\frac{eB}{T_{delay}^m P_0 \mathcal{R}}} \cdot \frac{E}{1-v^2} \cdot \frac{\lambda}{n} \cdot \frac{p}{z} \cdot \frac{h^3}{\left(R_m{}^2 - r_{min}{}^2\right)^2}$$

Wherein: m represents the number of membranes, e represents the fundamental charge, B represents the bandwidth, Tdelay represents the transmission of the waveguide on one membrane, P0 represents the laser power, R represents the detector responsivity, E represents the Young's modulus, v represents the Posson ratio, λ represents the wavelength, n represents the waveguide effective index, p represents the waveguide heart-to-heart spacing, z represents the distance of the waveguide centre to the center of the membrane, h represents the membrane thickness, Rm represents the membrane radius, rmin represents the minimum waveguide bend radius.

As such it follows that routing the waveguide over a plurality of membranes can increase sensitivity. In addition, and in contrast to some known concepts, the waveguides in embodiments according to the present disclosure are routed over zones of the membrane that impart an opposite overall response.

In some embodiments, e.g. as depicted in FIGS 3A to 3C one of the primary photonic waveguide 1 and the auxiliary photonic waveguide 2 follows a comparatively more outward curve than the other.

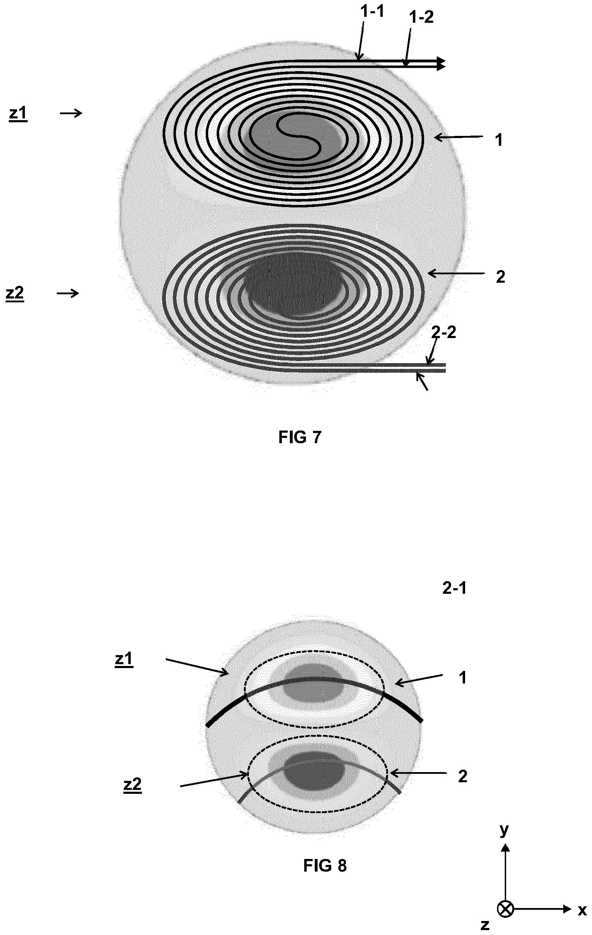

In other or further embodiments, e.g. as best illustrated by embodiments described in relation to FIGs 7 and 8, the zones imparting an opposite overall response can be understood as being positions having an out of phase deflection, e.g. an n times $\pi$ phase shift, in accordance with a higher harmonic resonance mode of the membrane.

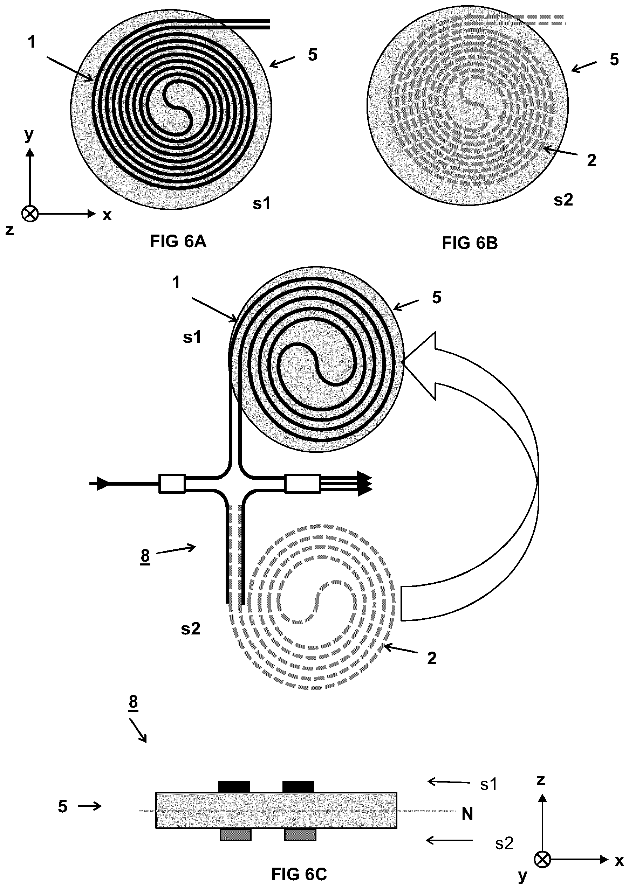

In yet other or further embodiments, best illustrated by embodiments described in relation to FIGs 6A to 6C the zones imparting an opposite overall response can be understood as being on opposite sides of the membrane.

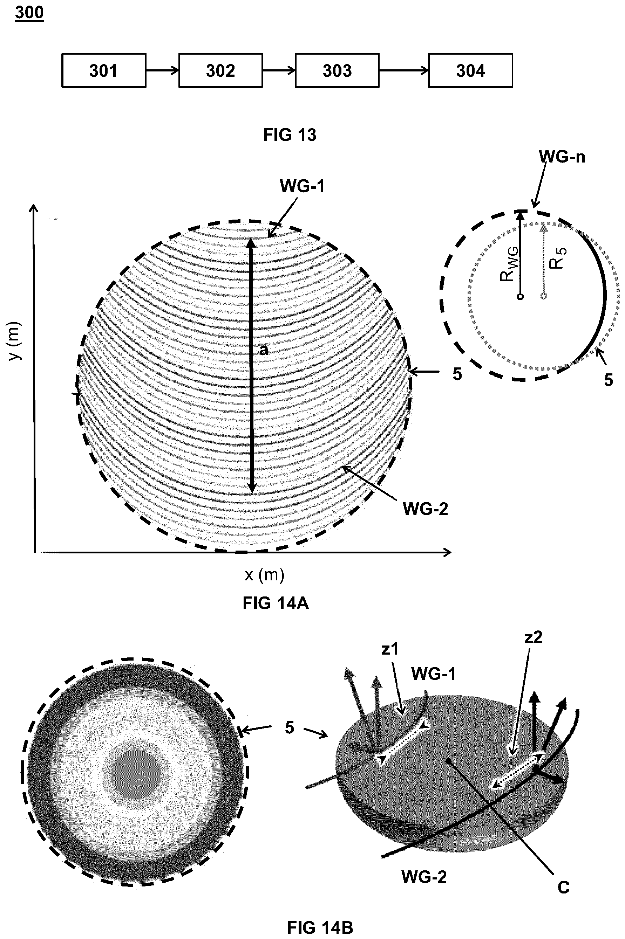

Turning to FIGs 14 to 17 the principle underlying the improved sensitivity as used in embodiments illustrated in some variations, e.g. as in FIG 3, is explained in more detail.

Specifically in FIG 14A a membrane 5 is shown in which a plurality of trajectories is indicated for curved waveguides WG-n (two of which are marked in the left image (WG-1 and WG-2). The WGs all have a radius of curvature greater than a radius of the membrane but with a different offset 'a'. FIG 14B illustrates that the relative position of the WGs on the membrane during primary mode resonance affects the overall contribution to overall contributions in the light path.

Specifically FIG 14B (left) illustrates, for a circular membrane, an out of plane deflection during a primary resonance mode. As indicated deflection is largest at the center of the membrane (red) and decreases to zero near the edges of the membrane (indicated by dashed line). As visualized in FIG 14B (right) waveguides (WG-1,WG-2) that curve along opposite sides of the membrane center (c) experience an opposite effect during membrane. Specifically, the waveguide (WG2) following an outer curve experiences an overall expansion as integrated over the length of the waveguide, whereas the more inward waveguide (WG1) experiences more compressive forces. It was found that a difference between length of the waveguides (dL) depends on an offset of the waveguides.

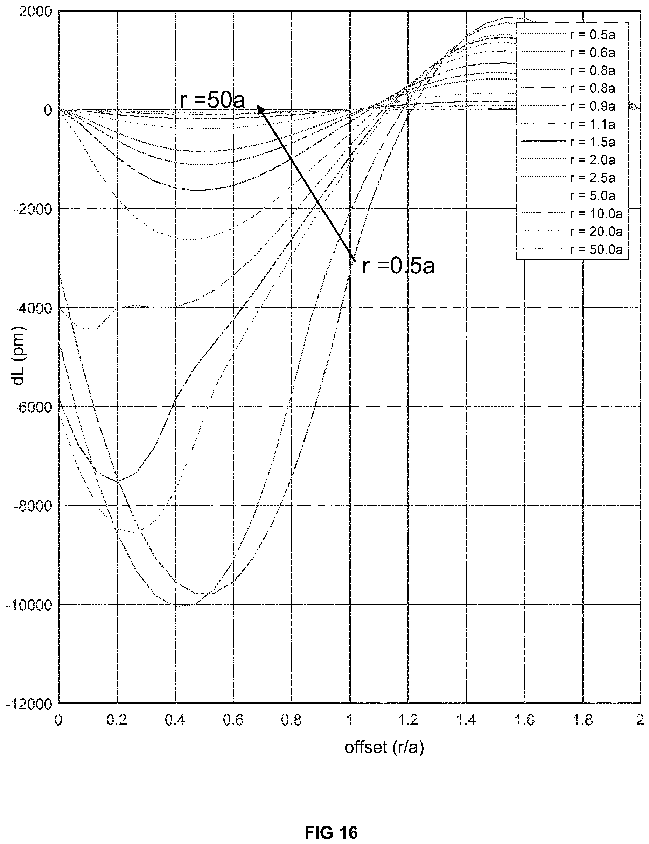

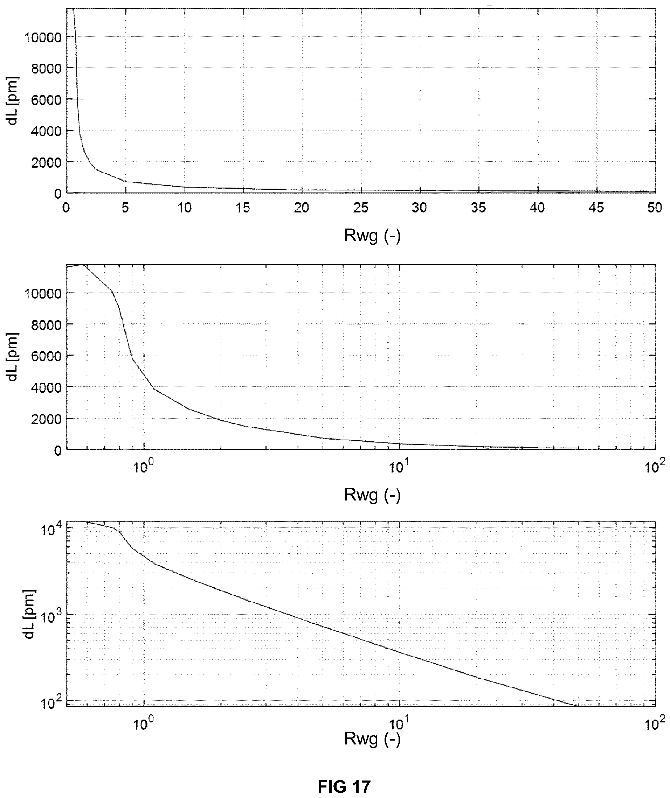

FIG 16 illustrates how dL (in picometers) of a curved waveguides varies with offset (r/a). Note that the results presented are for a membrane with radius ($R_5$) of 88 $\mu$m, and waveguides with a radius of curvature ($R_{WG}$) of 88 $\mu$m, for a n acoustic wave at a pressure of 1e+05 Pa. As indicated dL varies with offset (r/a) with a negative difference at low offsets and a positive dL at larger offsets. Note that for a negative pressure (-1e+05) the inverse behavior is found. As can be seen in Fig 17 a linear relation (on a log-log plot) was found between dL and the radius of the waveguide.

Turning to FIG 6 an embodiment is depicted wherein the first and second zones (z1,z2) are at opposing sides of the same membrane 5 across a neutral bending plane (N). Specifically, FIG 6A and B illustrate a membrane 5 having a primary photonic waveguide 1 disposed on a front side s1 and an auxiliary photonic waveguide 2 on a backside s2 of the same membrane. Optionally the waveguides can be disposed on a front, respectively a backside of different membranes. By depositing the primary and auxiliary photonic waveguides on opposing faces of a membrane the respective waveguides will experience opposing sign contraction/expansion during operation.

Figure 6C illustrates an embodiment of a transducer element according to a MZI configuration having one input and three outputs. The secondary waveguide (as positioned opposingly to the primary photonic waveguide) can be coupled to the multimode interference couplers (positioned in the same plane as the primary waveguide) by a so called vertical coupler element 8. This element vertically couples positions of waveguides that are in respective vertically separated layers. Specifically the coupler can comprise a section of a first waveguide and a section of the second waveguide portion, whereby the sections co-propagate with respect to each other forming an overlap (as seen in a plan view) thereby optically coupling the waveguides.

In some preferred embodiments, the first zone z1 and the second zone z2 are laterally spaced along the same side of the membrane at positions having an out of phase (e.g. an integer times $\pi$) deflection in accordance with a higher harmonic resonance mode of the membrane. The higher order mode can be a second order resonance mode. As illustrated in FIG 8 the zones having opposite sign deflection z1 z2 can be understood as being centered around loci having maximum deflection at the second order resonance.

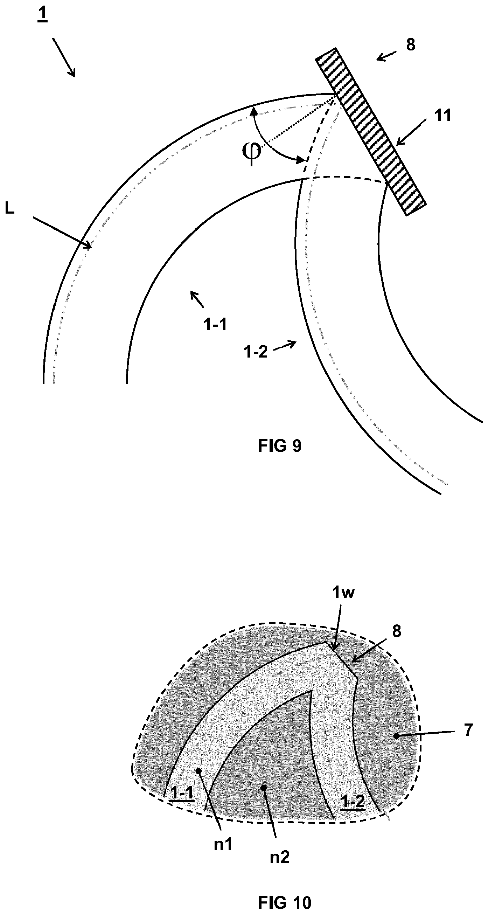

Depending on the dimension of the membrane (e.g. as based on an intendent operating frequency) the waveguides can follow an appropriate trajectory. In some embodiments, e.g. as shown in FIG 7, the waveguide can follow a spiraling trajectory. In other embodiments, (see e.g. FIG 8 and 10) the waveguides can follow a curved trajectory whereby a radius of curvature of the waveguide is larger than a radius of the membrane. The last variation can be particularly suitable for embodiments intended to operate with very small membranes (e.g. for operating at high resonance frequencies.

Of course the mechanisms as discussed above can be combined with variations wherein one or more of the waveguides extend along a plurality of membranes to further improve the sensitivity.

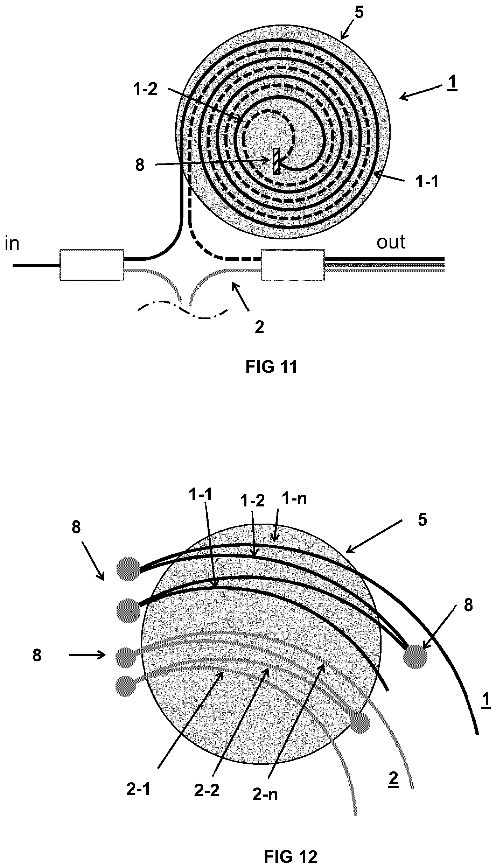

Alternatively, or in addition, one or more of the waveguides may be directed a plurality of times across individual membranes. Fig 12 illustrates a variation wherein the primary and auxiliary photonic waveguide 1,2 are routed across the membrane multiple times. For understanding each of the waveguides can be understood as being comprised of a plurality of segments that are interconnected by a coupler 8.

In a preferred embodiment, at least one of the primary photonic waveguide 1 and the auxiliary photonic waveguide 2 comprises a first curved waveguide section 1-1, 2-1 and a second curved waveguide section 1-2, 2-2 that are interconnected by a coupler 8, wherein the first waveguide section continues into the second waveguide section under an abrupt angle forming a merged interconnect, the interconnect terminating at a mirror element configured to reflect light traveling along one of the first and the second section to the other, forming the coupler. In a further preferred variation, the mirror element can be an upstanding sidewall of the waveguide, whereby the sidewall and waveguide potions are arranged to reflect a propagating wave by total internal reflection. Alternatively, the coupler may be a conventional coupler (e.g. an S-shaped section as shown in Fig 4B.

Turning to FIGs 9 and 10 the concept is described in more detail. Instead of two half-circles (S-shaped portion, cf. fig 4B), the light is redirected by means of a mirror 11. Two bent waveguides are terminated at the mirror with a mutual angle. If the condition of total internal reflection is satisfied the reflection can be essentially lossless. In practice, some optical loss can occur, e.g. due to a roughness at mirror or an off-vertical positioning and/or poor definition of the waveguide at the overlap (indicated by dashed lines). Note that the center of gravity of a light wave propagating in a curving waveguides tends to be near to the outer bend as opposed to the inner bend (indicated by the double dot-dashed line).

In a preferred embodiment, e.g. as shown in FIG 10 the mirror element is an upstanding sidewall 1w of the waveguide. The angle ($\theta$) is larger than two times a critical angle of incidence for total internal reflection.

$$\sin\frac{\varphi}{2} = \frac{n_{ambient}}{n_{eff}}$$

As indication, for waveguides formed of a refractive index (n1) of about 3.8 and a cladding with a refractive index (n2) of about 1.6 (e.g. polymers) an angle $\theta$ between the respective waveguides can be $\leq$ than about 60°. Very steep angles, e.g. < 20° can be less preferred because this would increase a dimension of the overlap. Alternatively or in addition the mirror element may be a sidewall of the waveguide having reflective coating (e.g. metal).

Similar as for the conventional S-shaped turn the merged interconnect is preferably provided at a central position of the membrane. Accordingly, in some variations both the first waveguide section and the second waveguide section, each comprise a spiral portion, such as a spiral represented by an Archimedean spiral, an involute of a circle, or a Fermat's spiral.

Turning to figure 13 a method of manufacturing an opto-acoustic transducer is described. The method comprises the steps of:

    providing (301) a membrane extending over an aperture in a surface of a carrier substrate, the membrane layer configured to deflect in response to an acoustic pressure wave (P);
    disposing (304) a primary photonic waveguide and an auxiliary photonic waveguide at the membrane
    wherein the primary and the auxiliary photonic waveguide are disposed along a respective curved primary and auxiliary curved trajectory over the membrane which extend over a respective first zone of the membrane and a respective second zone of the membrane providing opposite sign expansion/contraction due to a deflection of the membrane in response to the acoustic pressure wave.

In a preferred embodiment, the method further comprises a step of determining (302) one or more of a physical characteristic of the first and the second trajectory with respect to each other and the membrane prior step 304. The characteristic can include one or more of a size, shape, position and orientation of the waveguide with respect to each other and/or with respect to the membrane. For example, determining 302 can be realized by prior modelling response characteristics of the primary and auxiliary waveguide, e.g. by a finite element modelling process, comprising: maximizing a pathlength difference (dL) between the primary and auxiliary waveguide. As shown in FIG 16, the pathlength difference can be suitably optimized by integrating local contributions to contraction/expansions over the length of the waveguide due to the deflection of the membrane

In another or further preferred embodiment, the method can comprise a step of fixing 303 an orientation of a second or higher order harmonic resonance mode of the membrane. Step 303 can comprise one or more of: providing an anisotropic pre-stress to the membrane; forming the membrane of a layer having a crystalline anisotropy; and/or providing the membrane in an array of membranes whereby a spacing between adjacent membranes along orthogonal directions within the array differs by an amount providing directional mode coupling of second or higher order harmonic resonance modes of the membranes.

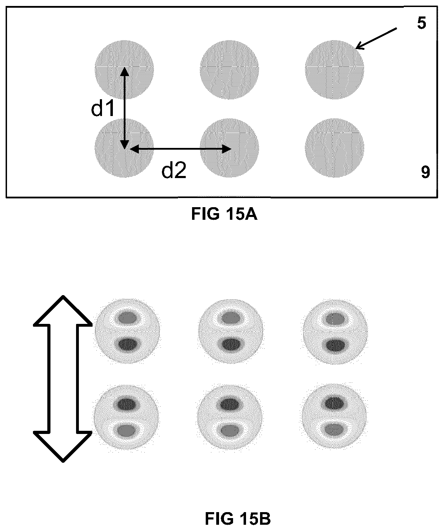

FIG 15A and 15B provide details at to a realization of mode coupling by providing an array of membranes with an anisotropic spacing. In some embodiments, preferential mode coupling can be obtained whereby higher order resonance modes of the membranes line up along a predefined direction, e.g. along the direction indicated by the arrow in FIG 15B.

Preferential mode coupling, e.g. as indicated, can be obtained by providing one or more of a prestress, crystalline anisotropy , or anisotropic spacing. For example, in the configuration shown in FIG 15A spacing between adjacent membranes (d1) along a direction differs from a spacing (d2) in a direction orthogonal thereto. Absolute and relative values of spacings can be determined using published methods. In general the spacing along different directions d1-d2) is larger than about 0.1 times a radius of the membranes.

For the purpose of clarity and a concise description, features are described herein as part of the same or separate embodiments, however, it will be appreciated that the scope of the invention may include embodiments having combinations of all or some of the features described. Of course, it is to be appreciated that any one of the above embodiments or processes may be combined with one or more other embodiments or processes to provide even further improvements in finding and matching designs and advantages.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An on-chip integrated opto-acoustic transducer element (10) for converting an acoustic pressure wave (P) into a modulation of a property of light (L), the transducer element comprising:

   a membrane (5) extending over an aperture (6) or recess in a surface of a carrier substrate (9), the membrane configured to deflect in response to an acoustic pressure wave (P);
   a primary photonic waveguide (1) disposed at the membrane, and
   an auxiliary photonic waveguide (2) disposed at the membrane
   wherein the primary waveguide and the auxiliary photonic waveguide follow a respective curved primary and auxiliary curved trajectory over the membrane which extend over a respective first zone (z1) of the membrane and a respective second zone (z2) of the membrane providing opposite sign expansion/contraction to the due to a deflection of the membrane in response to the acoustic pressure wave.

2. The transducer element according to claim 1, wherein the primary and auxiliary curved trajectory each have a radius of curvature larger than a radius of curvature of the membrane.

3. The transducer element according to any of the preceding claims, wherein the first and second zones (z1,z2) are at opposing sides of the membrane (5) across a neutral bending plane (N).

4. The transducer element according to any of the preceding claims, wherein the first zone (z1) and the second zone (z2) are laterally spaced along the same side of the membrane (5) at positions having an out of phase deflection in accordance with a higher harmonic resonance mode of the membrane.

5. The transducer element according to any of the preceding claims, wherein at least one of the primary curved trajectories and auxiliary curved trajectories extend along a plurality of the membrane, each membrane having a lateral dimension smaller than a maximum radius of curvature of the primary curved and auxiliary curved trajectories at the membrane.

6. The transducer element according to the preceding claim, wherein the plurality of the membrane is organized in an area smaller than half times the wavelength ($\lambda p$) of the acoustic pressure wave squared ($\lambda p/2$)$^2$.

7. The transducer element according to any of the preceding claims, wherein:

the membrane has an anisotropic pre-stress;

the membrane has a crystalline anisotropy; and/or

the membrane is comprised in an array of membranes whereby a spacing (d1,d2) between adjacent membrane along orthogonal directions within the array differs by an amount providing directional coupling of second or higher order harmonic resonance modes of the membranes.

8. The transducer element according to any of the preceding claims, wherein at least one of the primary photonic waveguide (1) and the auxiliary photonic waveguide (2) comprises a first curved waveguide section (1-1, 2-1) and a second curved waveguide section (1-2, 2-2) that are interconnected by a coupler (8), wherein the first waveguide section continues into the second waveguide section under an abrupt angle forming a merged interconnect, the interconnect terminating at a mirror element configured to reflect light traveling along one of the first and the second section to the other, forming the coupler.

9. The transducer element according to any of the preceding claims, wherein the primary photonic waveguide and the auxiliary photonic waveguide are spiral shaped, each having a first spiral section (1-1, 2-1) and a second spiral waveguide section (1-2, 2-2) that spiral inwardly, respectively outwardly, about a center of the first zone, respectively the second zone.

10. The transducer element according to claim 9, wherein the first spiral and the second spiral waveguide section are interconnected by the coupler according to claim 8.

11. An on-chip ultrasound receiver device (100) comprising:

the on-chip opto-acoustic transducer element (10) according to any of the preceding claims 1-10,

at least one light input (20) and one or more light output (30) operably connected to the primary photonic waveguide and the auxiliary photonic waveguide, and

readout circuitry (40) including one or more light detector (41) operably connected to the one or more light output and configured to provide as, electrical signals (S), an output based on a detected light intensity and/or phase shift.

12. An ultrasound imager system (200) comprising:

the on-chip ultrasound receiver element (100) according to claim 11, an ultrasound transmitter (150)

a light source (110) operably connected to the one or more on-chip ultrasound receiver element,

a processing unit (120) configured for controlling the ultrasound source, photo acoustic receiver, the light source, and the readout circuitry, and comprising a signal processor for construction an image using the output of the readout circuitry.

13. A method (300) of manufacturing an opto-acoustic transducer element for converting an acoustic pressure wave into a modulation of a property of light in the transducer element, the method comprising

providing (301) a membrane extending over an aperture or recess in a surface of a carrier substrate, the membrane layer configured to deflect in response to an acoustic pressure wave (P);

disposing (304) a primary photonic waveguide and an auxiliary photonic waveguide at the membrane

wherein the primary and the auxiliary photonic waveguide are disposed along a respective curved primary and auxiliary curved trajectory over the membrane which extend over a respective first zone of the membrane and a respective second zone of the membrane providing opposite sign expansion/contraction due to a deflection of the membrane in response to the acoustic pressure wave.

14. The method according to claim 13, further comprising a step of determining (302) a position, size, and/or a relative orientation of the primary photonic waveguide and an auxiliary photonic waveguide with respect to the membrane prior to disposing.

15. The method according to claim 13 or 14, further comprising a step of fixing (303) an orientation of a second or higher order harmonic resonance mode of the membrane by one or more of:

providing an anisotropic pre-stress to the membrane;

forming the membrane of a layer having a crystalline anisotropy; and/or

providing the membrane in an array of membranes whereby a spacing between adjacent membrane along orthogonal directions within the array differs by an amount providing directional coupling of second or higher order harmonic resonance modes of the membranes.

**FIG 1**

**FIG 2A**

**FIG 2B**

FIG 3A

FIG 3B

FIG 3C

FIG 4A

FIG 4B

FIG 5

1

5

y

z x

s1

**FIG 6A**

5

2

s2

**FIG 6B**

1

s1

5

8

s2

2

8

5

s1

N

s2

z

y x

**FIG 6C**

FIG 7

FIG 8

FIG 9

FIG 10

**FIG 11**

**FIG 12**

<u>300</u>

FIG 13

FIG 14A

FIG 14B

**FIG 16**

**FIG 17**

**FIG 15A**

**FIG 15B**

| | Europäisches Patentamt European Patent Office Office européen des brevets | **EUROPEAN SEARCH REPORT** | Application Number EP 23 18 4785 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/065945 A1 (VAN DER HEIDEN MAURITS SEBASTIAAN [NL] ET AL) 2 March 2023 (2023-03-02) * paragraphs [0114] – [0128]; figure 3 * ----- | 1-15 | INV. G01H9/00 A61B5/00 G01N21/17 G02B6/125 |
| A | US 2011/187868 A1 (CHANG CHIENLIU [JP] ET AL) 4 August 2011 (2011-08-04) * paragraph [0036]; figures 1A, 1B * ----- | 1-15 | |
| A | US 11 408 764 B2 (IMEC VZW [BE]; UNIV LEUVEN KATH [BE]) 9 August 2022 (2022-08-09) * column 28, line 35 – column 29, line 26; figures 8-9 * ----- | 1-15 | |
| A | US 2021/108978 A1 (HOFRICHTER JENS [NL]) 15 April 2021 (2021-04-15) * paragraphs [0007], [0059]; figures 5, 6 * ----- | 1-15 | |

TECHNICAL FIELDS SEARCHED (IPC)

G01H

The present search report has been drawn up for all claims

| Place of search | Date of completion of the search | Examiner |
|---|---|---|
| The Hague | 14 December 2023 | Naujoks, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 4785

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2023

| Patent document cited in search report | | | Publication date | Patent family member(s) | | | Publication date |
|---|---|---|---|---|---|---|---|
| US 2023065945 | A1 | | 02-03-2023 | EP | 3851816 | A1 | 21-07-2021 |
| | | | | EP | 4090922 | A1 | 23-11-2022 |
| | | | | US | 2023065945 | A1 | 02-03-2023 |
| | | | | WO | 2021145766 | A1 | 22-07-2021 |
| US 2011187868 | A1 | | 04-08-2011 | JP | 2009053031 | A | 12-03-2009 |
| | | | | US | 2011187868 | A1 | 04-08-2011 |
| | | | | WO | 2009028701 | A2 | 05-03-2009 |
| US 11408764 | B2 | | 09-08-2022 | EP | 3663817 | A1 | 10-06-2020 |
| | | | | US | 2020173843 | A1 | 04-06-2020 |
| US 2021108978 | A1 | | 15-04-2021 | CN | 112368556 | A | 12-02-2021 |
| | | | | EP | 3557211 | A1 | 23-10-2019 |
| | | | | JP | 7009650 | B2 | 25-01-2022 |
| | | | | JP | 2021521436 | A | 26-08-2021 |
| | | | | US | 2021108978 | A1 | 15-04-2021 |
| | | | | WO | 2019201800 | A1 | 24-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3851815 A1
- WO 2021145766 A1
- US 2020319019 A1

**Non-patent literature cited in the description**

- **S.M. LEINDERS et al.** *Nature Scientific Reports*, 2015, 14328